# EUROPÄISCHE PATENTSCHRIFT

(11) **EP 2 704 792 B1**
(45) Veröffentlichungstag und Bekanntmachung des Hinweises auf die Patenterteilung: **10.06.2015**
(21) Anmeldenummer: 12719576.6
(22) Anmeldetag: 25.04.2012
(51) Int. Cl.: A61N 1/36

(54) **VORRICHTUNG ZUR KOMBINIERTEN AUFBRINGUNG EINES TRANSKUTANEN ELEKTRISCHEN STIMULATIONSREIZES UND ABGABE EINES AKUSTISCHEN SIGNALS**
DEVICE FOR THE COMBINED APPLICATION OF A TRANSCUTANEOUS ELECTRICAL STIMULUS AND EMISSION OF AN ACOUSTIC SIGNAL
DISPOSITIF POUR L'APPLICATION D'UN STIMULUS ÉLECTRIQUE TRANSCUTANÉ COMBINÉE À L'ÉMISSION D'UN SIGNAL ACOUSTIQUE

(30) Priorität: 30.04.2011 DE 102011100065
(43) Veröffentlichungstag der Anmeldung: 12.03.2014
(73) Patentinhaber: Cerbomed GmbH, 91052 Erlangen (DE)
(72) Erfinder: BECK, Christoph, 91096 Möhrendorf (DE); ELLRICH, Jens, 91094 Langensendelbach (DE); HARTLEP, Andreas, 83607 Holzkirchen (DE); FRENKEL, Wolf Gerhard, 72514 Inzigkofen-Engelswies (DE)
(74) Vertreter: Gosdin, Michael
(86) Internationale Anmeldenummer: PCT/EP2012/001760
(87) Internationale Veröffentlichungsnummer: WO 2012/150009

(56) Entgegenhaltungen:
- US-A1- 2005 020 873
- US-A1- 2006 064 139
- US-A1- 2006 122 675
- US-A1- 2008 021 517
- US-A1- 2008 249 594
- US-B1- 6 198 971

## Beschreibung

Die Erfindung betrifft eine Vorrichtung zur kombinierten Aufbringung eines transkutanen elektrischen Stimulationsreizes auf die Oberfläche eines Abschnittes des menschlichen Ohrs und Abgabe eines akustischen Signals in den Gehörgang des Ohrs, wobei die Vorrichtung mindestens einen Elektrodenkopf mit mindestens einer Elektrode zur Aufbringung des elektrischen Stimulationsreizes aufweist, wobei die Vorrichtung einen Lautsprecher und einen Ausgabekanal für akustische Signale in den Gehörgang aufweist und wobei die Vorrichtung weiterhin eine Steuerungseinrichtung umfasst, mit der die Aufbringung des elektrischen Stimulationsreizes und die Abgabe akustischer Signale gesteuert werden kann.

Eine Vorrichtung dieser Art ist aus der DE 10 2006 036 069 B4 bekannt. Die dort beschriebene Vorrichtung in Form eines audiologischen Übertragungssystems sieht eine Möglichkeit der transkutanen Nervenstimulation vor, insbesondere des Vagusnervs, wobei gleichzeitig auch die Aufgabe eines akustischen Signals möglich ist; im genannten Falle ist primär, aber nicht ausschließlich an die Funktion eines Hörgeräts gedacht. Zur transkutanen Nervenstimulation ist ein bügelförmiger Fortsatz vorgesehen, der in den Gehörkanal des Ohrs eingeführt wird. Am Ende des Fortsatzes ist ein Elektrodenkopf mit zwei Elektroden vorgesehen.

Andere Lösungen werden in der US 2006/0064139 A1, in der US 2008/0249594 A1**,** in der US 2006/0122675 A1**,** in der US 2005/0020873 A1 und in der US 6 198 971 B1 beschrieben.

Es hat sich gezeigt, dass für manche Anwendungsfälle die hiermit mögliche Nervenstimulation noch nicht ausreichend bzw. ungünstig ist. Hier sei beispielsweise und insbesondere auf die Behandlung von Tinnitus hingewiesen, bei der grundsätzlich vorteilhafte Ergebnisse erzielt werden können, wenn die transkutane Nervenstimulation mit der gezielten Beaufschlagung mit akustischen Signalen kombiniert erfolgt.

Die transkutane Nervenstimulation als solche ist bereits in der DE 10 2006 023 824 B4 beschrieben. Hier ist eine Vorrichtung zur transkutanen Stimulation des Vagusnervs des menschlichen Körpers offenbart, die in diesem konkreten Falle in der Pinna des Ohres angeordnet werden kann. Die transkutane Stimulation des Vagusnervs erfolgt hier durch Kontaktierung des zu stimulierenden Gewebes mittels zweier kugelförmiger Elektroden, die elastisch gegen die Hautoberfläche verspannt werden.

Der vorliegenden Erfindung liegt daher die Aufgabe zugrunde, eine Vorrichtung der eingangs genannten Art bereitzustellen, die es in verbesserter Weise erlaubt, eine optimale Behandlung von verschiedenen Krankheiten, insbesondere von Tinnitus, zu ermöglichen, bei denen eine kombinierte Aufbringung von transkutaner Nervenstimulation und Abgabe von akustischen Signalen erfolgversprechend ist.

Die Lösung dieser Aufgabe durch die Erfindung ist dadurch gekennzeichnet, dass die mindestens eine Elektrode oder der sie tragende Elektrodenkopf ausgebildet ist, um in der Cymba conchae des Ohrs angeordnet werden zu können, dass am Elektrodenkopf ein elastischer Haltebügel angeordnet ist, der zum Umgreifen des Ohrs ausgebildet ist, und dass die Vorrichtung weiterhin einen Schwingungsgenerator aufweist, mit dem mechanische Schwingungen auf einen Teil des knöchernen Schädels aufgebracht werden können, wobei der Schwingungsgenerator am Haltebügel angeordnet oder in diesen integriert ist.

Es hat sich gezeigt, dass die Applikation transkutaner Stimulationsreize insbesondere im Bereich der Cymba conchae vorteilhaft ist, wenn dies mit der Applikation akustischer Signale kombiniert wird. Der Bereich der Cymba conchae ist dabei der Bereich der Concha des Ohres, der oberhalb des Crus helicis liegt; er wird auch als Hemiconcha superior bezeichnet. Unterhalb des Crus helicis nach unten erstreckt sich dann der Bereich des Cavum conchae.

Der Elektrodenkopf ist bevorzugt samt der mindestens einen Elektrode nierenförmig ausgebildet und weist eine weitgehend ebene Auflagefläche an der dem Ohr zugewandten Seite auf. Dabei ist besonders bevorzugt vorgesehen, dass der Elektrodenkopf die Oberfläche des Ohres über eine Kontaktfläche kontaktiert, die mindestens 50 % der Oberfläche der Cymba conchae des Ohrs abdeckt; ein weiter bevorzugter Bereich beträgt sogar mindestens 80 % der Oberfläche der Cymba conchae.

Der Ausgabekanal für akustische Signale ist bevorzugt als Fortsatz am Elektrodenkopf ausgebildet.

Mit dem Schwingungsgenerator können insbesondere mechanische Schwingungen auf das Felsenbein oder das Mastoid aufgebracht werden. Hiermit sind die beiden Hörtests nach Weber und Rinne (s. unten) durchführbar.

Die Steuerungseinrichtung kann ausgebildet sein, zeitlich abgestimmt elektrische Stimulationsreize über die Elektroden und akustische Signale über den Lautsprecher und gegebenenfalls mechanische Schwingungen über den Schwingungsgenerator zu veranlassen, wobei die zeitliche Abstimmung vorzugsweise Simultanphasen, zeitversetzte Phasen und teilweise überlappende Phasen von Stimulationsreiz, Signal und gegebenenfalls mechanischen Schwingungen umfasst. Die Steuerungseinrichtung weist hierfür bevorzugt ein elektronisches Modul für die Veranlassung von elektrischen Stimulationsreizen, akustischen Signalen und gegebenenfalls mechanischen Schwingungen auf.

Die mindestens eine Elektrode besteht nach einer Weiterbildung zumindest teilweise aus einem Kunststoffmaterial, das mit Mitteln zur Herstellung elektrischer Leitfähigkeit versehen ist. Diese Mittel zur Herstellung elektrischer Leitfähigkeit können elektrisch leitfähige Partikel sein, die in das Kunststoffmaterial eingelagert sind; die Mittel können auch mindestens eine galvanisch auf einen Grundkörper der Elektrode aufgebrachte elektrisch leitfähige Metallschicht sein.

Ferner können mindestens zwei Elektroden vorgesehen sein, nämlich mindestens eine Stimulationselektrode und eine Referenzelektrode, wobei die mindestens eine Stimulationselektrode die Oberfläche des Ohres über eine erste Kontaktfläche kontaktiert und wobei die mindestens eine Referenzelektrode die Oberfläche des Ohres über eine zweite Kontaktfläche kontaktiert, wobei die zweite Kontaktfläche mindestens 3 mal so groß ist, vorzugsweise mindestens 5 mal so groß ist, wie die erste Kontaktfläche.

Eine alternative Ausgestaltung der Erfindung sieht eine Vorrichtung zur kombinierten Aufbringung eines transkutanen elektrischen Stimulationsreizes auf die Oberfläche eines Abschnittes des menschlichen Ohrs und die Abgabe einer mechanischen Schwingung auf einen Teil des knöchernen Schädels, bevorzugt auf das Felsenbein oder das Mastoid, vor, wobei die Vorrichtung mindestens einen Elektrodenkopf mit mindestens einer Elektrode zur Aufbringung des elektrischen Stimulationsreizes aufweist, wobei die Vorrichtung einen Schwingungsgenerator zur Erzeugung mechanischer Schwingungen aufweist, wobei die Vorrichtung weiterhin eine Steuerungseinrichtung umfasst, mit der die Aufbringung des elektrischen Stimulationsreizes und die Abgabe mechanischer Schwingungen gesteuert werden kann, und wobei die mindestens eine Elektrode oder der sie tragende Elektrodenkopf ausgebildet ist, um in der Cymba conchae des Ohrs angeordnet werden zu können. Demgemäß besteht die akustische Anregung also lediglich aus dem Schwingungsgenerator.

Der erwähnte Elektrodenkopf hat zwar bevorzugt zwei Elektroden, nämlich eine Stimulation- und eine Referenzelektrode (wie in den nachfolgenden Ausführungsbeispielen dargestellt). Allerdings kann grundsätzlich auch mit nur einer einzigen (Stimulation)Elektrode am Elektrodenkopf gearbeitet werden; in diesem Falle ist eine zweite (Referenz)Elektrode an einem anderen Ort angeordnet, beispielsweise eine neutrale bzw. indifferente Gegenelektrode in einem anderen Bereich des Ohrs, generell aber auch an einem anderen Körperteil, z. B. am Unterschenkel.

Erfindungsgemäß wird also insbesondere angestrebt, dass tonaler Tinnitus (insbesondere Pfeif-, Summ- oder Zirptöne) durch die Kombination von transkutaner Vagusnervstimulation und Tönen behandelt wird. Dies umfasst auch die Behandlung von Tinnitus mit anderen Qualitäten, wie Klingeln, Rauschen, Maschinengeräuschen, Brummen, Zischen, Knacken, Klopfen, entweder gleichbleibend oder rhythmisch-pulsierend, teilweise auch pulssynchron.

Bei der Behandlung von Tinnitus erfolgt zumeist zunächst die Bestimmung der Tonhöhe des Ohrgeräusches (Vergleichsmessung mit Sinustönen oder Schmalbandgeräuschen). Weiterhin kann eine Verdeckungsmessung mit Sinustönen oder Schmalbandgeräuschen erfolgen. Typischerweise kann hierbei ein innenohrbedingtes Ohrgeräusch durch Sinustöne oder Schmalbandgeräusche mit einem Schalldruckpegel von 5 bis 10 dB (bis 20 dB) über der Schwelle verdeckt werden. Weiterhin kann die Messung der Residual-Inhibition erfolgen. Typisch für innenohrbedingte Ohrgeräusche ist, dass das Ohrgeräusch nach Beendigung einer Verdeckung mit Sinustönen oder Schmalbandgeräuschen einige Sekunden unterdrückt wird und erst dann wieder auftritt.

Wenn die Frequenz-Bandbreite eines individuellen Ohrgeräusches objektiv mit hinreichender Genauigkeit bestimmt ist, kann dieses Geräusch mit einem Frequenzgenerator (Lautsprecher) künstlich nachgebaut und dem Patienten die hiervon ausgesparten Frequenzen zusammen mit einer transkutanen Vagusnervstimulation angeboten werden.

Mögliche Behandlungsweisen sind hierbei ein periodisches An- und Abschwellen der Lautstärke des angebotenen Tones oder Frequenzgemisches, beispielsweise in Sinusform.

Möglich ist auch ein sequenziell-alternierender, teilweise überlappender Einsatz einer Tontherapie und der transkutanen Vagusnervstimulation mit dem Ziel, einen Umlerneffekt des Gehirns zu optimieren.

Weiterhin kann eine spezielle Sequenz mit Applikation der als wirksam identifizierten Tonfrequenzen über das betroffene Ohr und auch über das nicht (so stark) betroffene Ohr zur Ausnutzung von sog. Crossover-Effekten erfolgen.

Ferner kann eine pulsierte Applikation der Tonfrequenzen erfolgen, entweder simultan zur ebenfalls gepulsten transkutanen Vagusnervstimulation, alternierend in den jeweiligen Lücken der ebenfalls gepulsten transkutanen Vagusnervstimulation oder simultan zur gleichmäßig verabreichten transkutanen Vagusnervstimulation.

Eine pulsierte Applikation der transkutanen Vagusnervstimulation kann erfolgen entweder simultan zur ebenfalls gepulsten Tontherapie, alternierend in den jeweiligen Lücken der ebenfalls gepulsten Tontherapie oder simultan zur gleichmäßig verabreichten Tontherapie.

Bei komplexen Geräuschen kann auch anstelle der simultanen Applikation des gesamten Frequenzbandes ein dieses Frequenzband sinusartig über mehrere Sekunden hinweg abdeckender Frequenzdurchgang von der höchsten bis zur tiefsten identifizierten Ohrgeräusch-Frequenz vorgesehen werden.

Als spezielle Anwendung ist auch die Hinzunahme einer transkraniellen Magnetstimulation als Verstärker für den Retrainings-Effekt der kombinierten transkutanen Vagusnervstimulation mit Tönen möglich.

Hierbei kann eine Beschreibung eines über mehrere Tage bzw. Wochen angelegten Retrainingsprogramms erfolgen mit einem von Tag zu Tag bzw. von Sitzung zu Sitzung individuell für den Patienten getestetem bzw. festgelegtem Stimulationsmuster, beispielsweise mit genau definierten Stepup und Step-down-Abschnitten.

In der Zeichnung sind Ausführungsbeispiele der Erfindung dargestellt. Es zeigen:
- Fig. 1: eine Ohrmuschel (Pinna) eines Menschen,
- Fig. 2: die Ohrmuschel mit einer Vorrichtung zur Aufbringung eines elektrischen Stimulationsreizes und zur Abgabe eines akustischen Signals und
- Fig. 3: in perspektivischer Darstellung eine zu Fig. 2 alternativ ausgebildete Vorrichtung zur Aufbringung eines elektrischen Stimulationsreizes und zur Abgabe eines akustischen Signals.

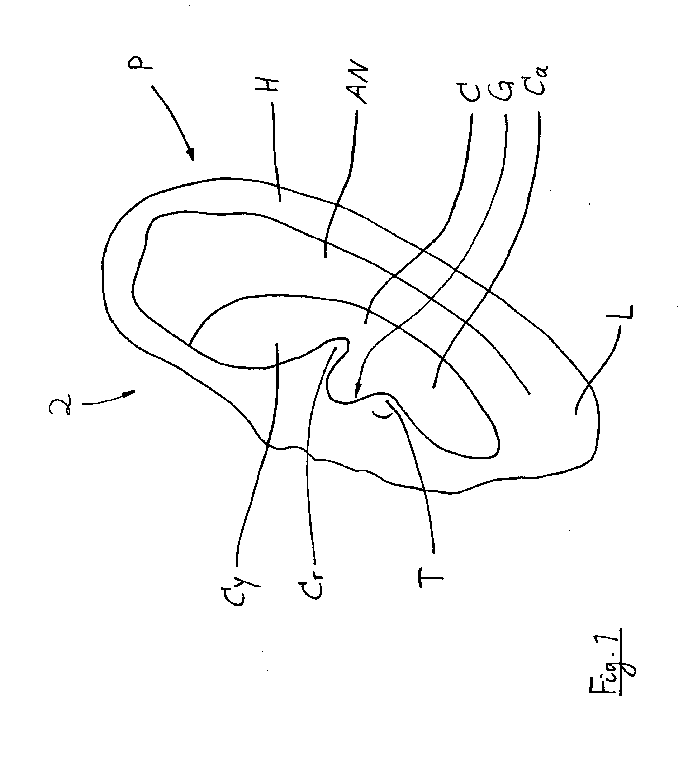

In Fig. 1 ist ein (Außen)Ohr 2 eines Menschen skizziert, dessen Form durch die Pinna (Ohrmuschel) P definiert ist. Die Pinna P umfasst in bekannter Weise die Helix H und Antihelix AN; zentral ist die Concha C angeordnet, die seitlich vom Tragus T begrenzt wird. Im unteren Bereich befindet sich die Lobule L. Die Concha C unterteilt sich in einen oberen und einen unteren Bereich; beide Bereiche werden durch die Crus helicis Cr voneinander abgetrennt. Der obere Teil der Concha C ist die Cymba conchae Cy, der untere Teil ist das Cavum conchae Ca. Die Lage des Gehörgangs ist mit G markiert.

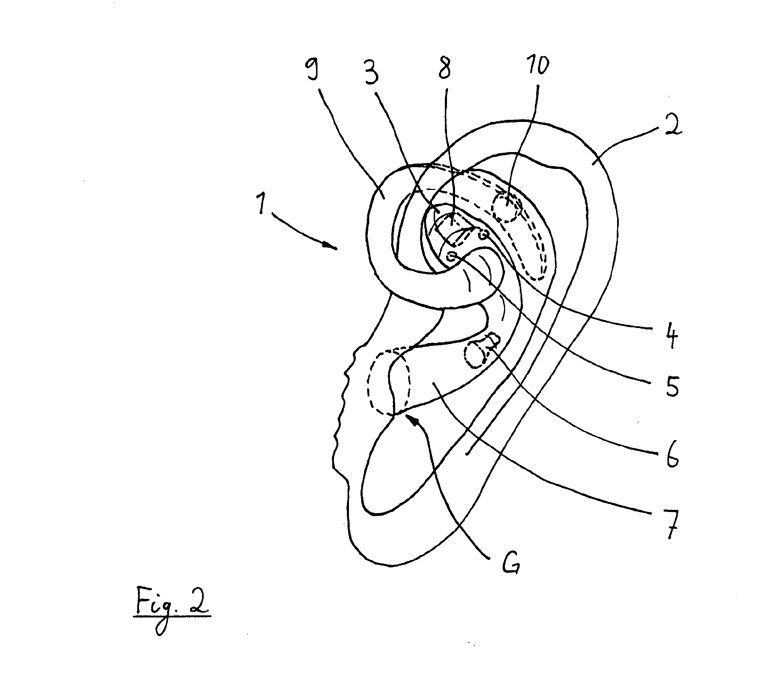

In der Pinna P ist - wie es in Fig. 2 gesehen werden kann - eine Vorrichtung 1 angeordnet, mit der einerseits ein transkutaner elektrischer Reiz auf einen Teil der Oberfläche des Ohrs 2 ausgeübt werden kann und mit der andererseits ein akustisches Signal in den Gehörgang G abgegeben werden kann.

Was den prinzipiellen Aufbau einer Vagusnerv-Stimulationsvorrichtung anbelangt, entspricht der beschriebene Aufbau der vorbekannten Lösung gemäß der oben genannten DE 10 2006 023 824 B4 der Anmelderin, auf die insoweit ausdrücklich Bezug genommen wird.

Die Vorrichtung 1 ist ausgebildet, um im Bereich des Vagusnervs am Ohr der die Vorrichtung 1 benutzenden Person angebracht zu werden. Damit kann eine transkutane Stimulation des Vagusnervs vorgenommen werden.

Demgemäß hat die Vorrichtung 1 einen Elektrodenkopf 3 mit zwei Elektroden 4 und 5, nämlich mit einer Stimulationselektrode 4 und einer Referenzelektrode 5. Der Elektrodenkopf 3 hat eine nierenförmige Ausgestaltung und ist an seiner dem Ohr zugewandten Seite eben ausgebildet, so dass er so im Ohr 2 platziert werden kann, dass er auf der Cymba conchae Cy aufliegt. Wie aus der Zusammenschau der Figuren 1 und 2 gesehen werden kann, erfolgt dies so, dass der wesentliche Bereich der Cymba conchae Cy vom Elektrodenkopf 3 abgedeckt wird.

Die beiden Elektroden 4, 5 sind in Fig. 2 als kleine, punktförmige Kontakte angedeutet. Es ist auch möglich, dass die Elektrodenfläche sehr viel größer ist, wobei die Elektroden insbesondere durch zwei (oder auch mehrere) Areale definiert sein können, die sich flächig über die Cymba conchae ausdehnen.

Damit die Vorrichtung 1 sicher in der Pinna gehalten wird, ist am Elektrodenkopf 3 ein Haltebügel 9 angeformt. Der Haltebügel 9 ist vorliegend als elastisches, C-förmig ausgebildetes Teil ausgeführt und greift mit seinem Ende so hinter das Ohr 2, dass er auf der Ohrwurzel zu liegen kommt und so die Vorrichtung 1 in der Pinna hält.

Am Elektrodenkopf 3 ist weiterhin - als Fortsatz des Elektrodenkopfs ausgebildet - ein Ausgabekanal 7 für akustische Signale angeordnet, dessen Ende ausgebildet ist, um in den Gehörgang G eingeführt werden zu können. Im Ausgabekanal 7 ist an geeigneter Stelle ein Lautsprecher 6 angeordnet.

Der Elektrodenkopf 3 stellt gleichzeitig ein Gehäuse dar, in dem zumindest eine Steuerungseinrichtung 8 angeordnet ist, ebenfalls bevorzugt eine Stromversorgung (wiederaufladbare Batterie). Nicht dargestellt ist, dass es aber grundsätzlich auch möglich ist, die Steuerung und Energieversorgung extern vorzunehmen, wozu dann die Vorrichtung 1 ein Kabel aufweist, um von einer externen Steuerungsvorrichtung und Energiequelle versorgt zu werden.

Als weiteres Element umfasst die Vorrichtung 1 einen Schwingungsgenerator 10 für mechanische Schwingungen. Dieser Schwingungsgenerator ist vorliegend in den Haltebügel 9 integriert. Wird die Vorrichtung 1 im Ohr 2 angeordnet, kommt der Schwingungsgenerator 10 hinter dem Ohr im Bereich der Ohrwurzel zur Anlage. Demgemäß können mechanische Schwingungen direkt in das knöcherne Gewebe des Hirnschädels eingeleitet werden, so dass ein Ton in das im Felsenbein liegende Innenohr eingeleitet werden kann, der vom Patienten nicht über den Gehörgang G, sondern direkt über die Knochenleitung wahrgenommen werden kann.

Der Schwingungsgenerator 10 nutzt dieselben physiologischen Gesetzmäßigkeiten wie der sog. Weber-Versuch. Dieser Versuch ist eine Untersuchung zur Feststellung einer Lateralisation des Hörempfindens unter Verwendung einer Stimmgabel. Er ist zusammen mit dem Rinne-Versuch ein Standardtest der Hals-Nasen-Ohren-Heilkunde zur Untersuchung einer Hörstörung.

Beim Weber-Versuch wird der Fuß einer schwingenden Stimmgabel dem Patienten auf den Scheitel gesetzt. Der Schall wird über Knochenleitung phasengleich in beide Innenohren übertragen. Der Normalhörende hört den Ton der Stimmgabel in beiden Ohren gleich, er hat daher den Eindruck, diesen in der Mitte des Kopfes zu hören, der Ton wird nicht lateralisiert. Gibt der Patient indes an, den Ton nur auf einer Seite zu hören, liegt eine Lateralisierung vor, d. h. eine einseitige oder asymmetrische Hörstörung liegt vor.

Bei einer einseitigen Schallempfindungsstörung wird der Ton vom besser hörenden (normalen) Innenohr lauter wahrgenommen, der Patient lateralisiert also ins gesunde Ohr. Bei einer einseitigen Schallleitungsstörung wird indes der Ton im erkrankten Ohr lauter gehört, weil eine retrograde Schallabstrahlung nur über die gesunde Hörknöchelchen-Kette erfolgt.

Mit dem Weber-Versuch ist also bei einer einseitigen Hörstörung eine schnelle und verlässliche Unterscheidung zwischen Schallempfindungsstörung und Schallleitungsstörung möglich, also beispielsweise zwischen einem Hörsturz und einem Paukenerguss. Die Schalleinleitung erfolgt insbesondere über das Felsenbein per Knochenleitung, nicht über den äußeren Gehörgang (Meatus acusticus externus).

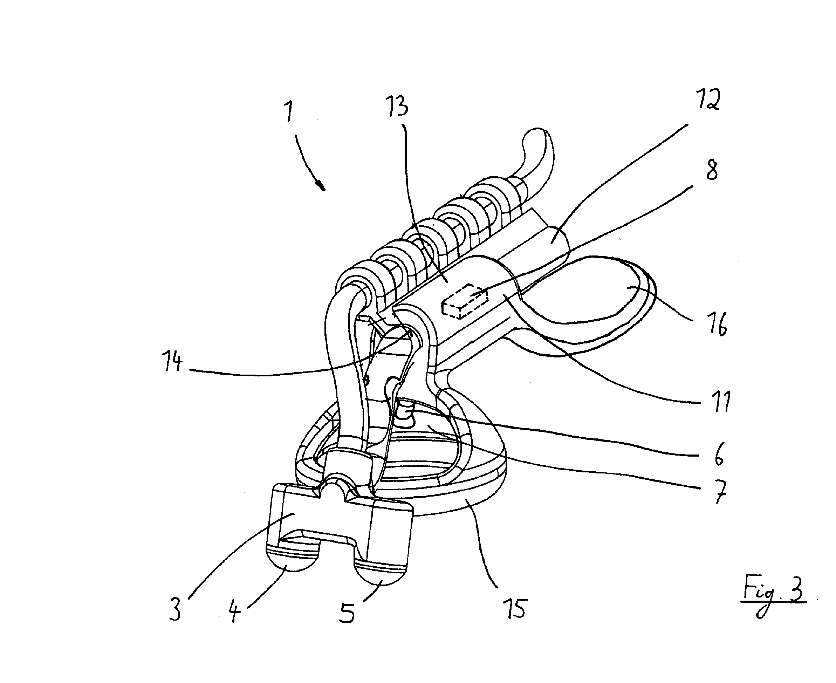

In Fig. 3 ist eine alternative Gestaltung der Vorrichtung 1 zu sehen, der aber auch das Prinzip zugrunde liegt, die transkutane Vagusnervstimulation im Bereich der Cymba conchae vorzunehmen, während neben der Nervenstimulation auch eine Beaufschlagung mit akustischen Signalen erfolgt. Demgemäß sind auch hier die oben genannten Komponenten der Vorrichtung 1 gegeben (nicht dargestellt ist hier der Schwingungsgenerator 10, der aber natürlich hier genauso vorgesehen werden kann).

Die Vorrichtung 1 hat als wesentliche Bauteile ein Halteelement 11 sowie eine Haltestange 12. Die Haltestange 12 trägt an einem axialen Ende den Elektrodenkopf 3, der mit zwei Elektroden 4, 5 versehen ist, nämlich mit einer Stimulationselektrode 4 und einer (baugleichen) Referenzelektrode 5. Das Halteelement 11 hat einen zentralen Abschnitt, der von einer Linearführung 13 dominiert wird. Hierbei handelt es sich um einen sich in eine Längsrichtung erstreckenden Materialabschnitt mit stabartiger Form, in den eine kreisförmige Ausnehmung 14 eingeformt ist.

Am vorderen Ende der Linearführung 13 ist ein Auflageteil 15 angeformt; am hinteren Ende ist eine Wangenauflage 16 angeformt. Der Auflageteil 15 weist einen ringförmigen Abschnitt auf; die Wangenauflage 16 ist vorliegend als flacher Abschnitt ausgeführt und dient als zusätzliche Stabilisierung der Auflage der Vorrichtung 1. Das Halteelement 11 ist mittels der Linearführung 13 in der Lage, die Haltestange 12 in Richtung der Längsachse linear zu verschieben.

Der Lautsprecher 6 liegt hier oberhalb des ringförmigen Auflageteils 15, so dass hier ein Ausgabekanal 7 in Form eines Schallelements gebildet wird, mit dem Töne des Lautsprechers 6 in den Gehörgang gelangen können. Das ringförmige Auslageteil 15 liegt bei angelegter Vorrichtung 1 genau über der Hörkanal-Öffnung. Der Lautsprecher 6 kann den gesamten Ring des Auflageteils 15 ausfüllen.

In beiden Ausführungsvarianten nach Fig. 2 und Fig. 3 sind gleich große Elektroden 4, 5 vorgesehen, die halbkugelförmig ausgeformt sein können. Möglich ist aber auch eine Variation hiervon. Die Stimulations- und Referenzelektrode können jeweils eine Form haben, die der Region bzw. Oberfläche des Ohrs 2 angepasst ist, wo sie platziert werden sollen. Es können hier ovale Strukturen oder nierenförmige Strukturen vorgesehen werden. Die mindestens zwei Elektroden kontaktieren die Oberfläche des Ohres 2 und namentlich der Cymba conchae mit jeweiligen Kontaktflächen. Dabei kann die eine Kontaktfläche erheblich größer sein als die andere. Versuche haben ergeben, dass vorteilhafte Effekte erreicht werden können, wenn die eine Fläche mindestens 3 mal so groß ist wie die andere Fläche. Die Elektroden 4, 5 sind dabei in einem Abstand angeordnet. Der minimale Abstand beträgt zumeist 5 mm. Es können aber auch Abstände bis zu 50 mm vorgesehen werden.

### Bezugszeichenliste:

- 1: Vorrichtung
- 2: Ohr
- 3: Elektrodenkopf
- 4: Elektrode
- 5: Elektrode
- 6: Lautsprecher
- 7: Ausgabekanal für akustische Signale (Schallelement)
- 8: Steuerungseinrichtung
- 9: Haltebügel
- 10: Schwingungsgenerator
- 11: Halteelement
- 12: Haltestange
- 13: Linearführung
- 14: kreisförmige Ausnehmung
- 15: Auflageteil
- 16: Wangenauflage

- G: Gehörgang
- Cy: Cymba conchae
- AN: Antihelix
- C: Concha
- Ca: Cavum conchae
- Cr: Crus helicis
- H: Helix
- L: Lobule
- P: Pinna
- T: Tragus

## Patentansprüche

1. Vorrichtung (1) zur kombinierten Aufbringung eines transkutanen elektrischen Stimulationsreizes auf die Oberfläche eines Abschnittes des menschlichen Ohrs (2) und Abgabe eines akustischen Signals in den Gehörgang (G) des Ohrs (2), wobei die Vorrichtung (1) mindestens einen Elektrodenkopf (3) mit mindestens einer Elektrode (4, 5) zur Aufbringung des elektrischen Stimulationsreizes aufweist, wobei die Vorrichtung (1) einen Lautsprecher (6) und einen Ausgabekanal (7) für akustische Signale in den Gehörgang (G) aufweist, wobei die Vorrichtung (1), weiterhin eine Steuerungseinrichtung (8) umfasst, die dazu ausgebildet ist, die Aufbringung des elektrischen Stimulationsreizes und die Abgabe akustischer Signalezusteuern,
und wobei die mindestens eine Elektrode (4, 5) oder der sie tragende Elektrodenkopf (3) ausgebildet ist, um in der Cymba conchae (Cy) des Ohrs (2) angeordnet werden zu können,
**dadurch gekennzeichnet,**
**dass** am Elektrodenkopf (3) ein elastischer Haltebügel (9) angeordnet ist, der zum Umgreifen des Ohrs (2) ausgebildet ist, und
**dass** die Vorrichtung (1) weiterhin einen Schwingungsgenerator (10) aufweist, der dazu ausgebildet ist, mechanische Schwingungen auf einen Teil des knöchernen Schädels aufbringen zu können, wobei der Schwingungsgenerator (10) am Haltebügel (9) angeordnet oder in diesen integriert ist.

2. Vorrichtung nach Anspruch 1, **dadurch gekennzeichnet, dass** der Elektrodenkopf (3) samt der mindestens einen Elektrode (4, 5) nierenförmig ausgebildet ist und eine weitgehend ebene Auflagefläche an der dem Ohr zugewandten Seite aufweist, wobei der Elektrodenkopf (3) die Oberfläche des Ohres (2) über eine Kontaktfläche kontaktiert, die mindestens 50 % der Oberfläche der Cymba conchae (Cy) des Ohrs (2) abdeckt.

3. Vorrichtung nach Anspruch 1 oder 2, **dadurch gekennzeichnet, dass** der Ausgabekanal (7) für akustische Signale als Fortsatz am Elektrodenkopf (3) ausgebildet ist.

4. Vorrichtung nach einem der Ansprüche 1 bis 3, **dadurch gekennzeichnet, dass** mit dem Schwingungsgenerator (10) mechanische Schwingungen auf das Felsenbein oder das Mastoid aufgebracht werden können.

5. Vorrichtung nach einem der Ansprüche 1 bis 4, **dadurch gekennzeichnet, dass** die Steuerungseinrichtung (8) ausgebildet ist, zeitlich abgestimmt elektrische Stimulationsreize über die Elektroden (4, 5) und akustische Signale über den Lautsprecher (6) und gegebenenfalls mechanische Schwingungen über den Schwingungsgenerator (10) zu veranlassen, wobei die zeitliche Abstimmung Simultanphasen, zeitversetzte Phasen und teilweise überlappende Phasen von Stimulationsreiz, Signal und Schwingungen umfasst.

6. Vorrichtung nach Anspruch 5, **dadurch gekennzeichnet, dass** die Steuerungseinrichtung (8) ein elektronisches Modul für die Veranlassung von elektrischen Stimulationsreizen, akustischen Signalen und mechanischen Schwingungen aufweist.

7. Vorrichtung nach einem der Ansprüche 1 bis 6, **dadurch gekennzeichnet, dass** die mindestens eine Elektrode (4, 5) zumindest teilweise aus einem Kunststoffmaterial besteht, das mit Mitteln zur Herstellung elektrischer Leitfähigkeit versehen ist, wobei die Mittel zur Herstellung elektrischer Leitfähigkeit elektrisch leitfähige Partikel sind, die in das Kunststoffmaterial eingelagert sind oder wobei die Mittel zur Herstellung elektrischer Leitfähigkeit mindestens eine galvanisch auf einen Grundkörper der Elektrode (4, 5) aufgebrachte elektrisch leitfähige Metallschicht sind.

8. Vorrichtung nach einem der Ansprüche 1 bis 7, **dadurch gekennzeichnet, dass** mindestens zwei Elektroden (4, 5) vorgesehen sind, nämlich mindestens eine Stimulationselektrode (4) und eine Referenzelektrode (5), wobei die mindestens eine Stimulationselektrode (4) die Oberfläche des Ohres (2) über eine erste Kontaktfläche kontaktiert und wobei die mindestens eine Referenzelektrode (5) die Oberfläche des Ohres (2) über eine zweite Kontaktfläche kontaktiert, wobei die zweite Kontaktfläche mindestens 3 mal so groß ist wie die erste Kontaktfläche.

## Claims

1. Device (1) for the combined application of a transcutaneous electrical stimulus to the surface of a portion of the human ear (2) and emission of an acoustic signal into the auditory canal (G) of the ear (2), wherein the device (1) comprises at least on electrode head (3) with at least one electrode (4, 5) for the application of the electrical stimulus, wherein the device (1) comprises a loudspeaker (6) and an output channel (7) for acoustic signals into the auditory canal (G), wherein the device (1) further comprises a control device (8) which is designed to control the application of the electrical stimulus and the emission of acoustic signals and wherein the at least one electrode (4, 5) or the electrode head (3) which carries it is designed to allow it to be arranged in the Cymba conchae (Cy) of the ear (2),
**characterized in**
**that** an elastic holding bow (9) is arranged at the electrode head (3) which is designed for encompassing the ear (2) and
**that** the device (1) further comprises a vibration generator (10) which is designed to apply mechanical vibrations onto a part of the osseous cranium, wherein the vibration generator (10) is arranged on or integrated within the holding bow (9).

2. Device according to claim 1, **characterized in that** the electrode head (3) with the at least one electrode (4, 5) is designed kidney-shaped and comprises a substantial plane resting surface at the side facing the ear, wherein the electrode head (3) contacts the surface of the ear (2) at a contact area which covers at least 50 % of the surface of the Cymba conchae (Cy) of the ear (2).

3. Device according to claim 1 or 2, **characterized in that** the output channel (7) for acoustic signals is designed as an extension at the electrode head (3).

4. Device according to one of claims 1 to 3, **characterized in that** by means of the vibration generator (10) mechanical vibrations can be applied onto the petrosal bone or onto the mastoid.

5. Device according to one of claims 1 to 4, **characterized in that** the control device (8) is designed to provoke chronologically agreed electrical stimuli via the electrodes (4, 5) and acoustical signals via the loudspeaker (6) and if applicable mechanical vibrations via the vibration generator (10), wherein the chronological agreement comprises simultaneous phases, time shifted phases and partially overlapping phases of electrical stimulus, signal and vibrations.

6. Device according to claim 5, **characterized in that** the control device (8) comprises an electrical module for the provoking of electrical stimuli, acoustic signals and mechanical vibrations.

7. Device according to one of claims 1 to 6, **characterized in that** the at least one electrode (4, 5) consists at least partially of a synthetic material, which material is supplied with means for the establishment of electrical conductibility, wherein the means for the establishment of electrical conductibility are electrical conductible particles, which particles are incorporated into the synthetic material or wherein the means for the establishment of electrical conductibility are at least one electrical conductible metal layer, which is applied galvanically on a base body of the electrode (4, 5).

8. Device according to one of claims 1 to 7, **characterized in that** at least two electrodes (4, 5) are provided, that is to say at least one stimulation electrode (4) and one reference electrode (5), wherein the at least one stimulation electrode (4) contacts the surface of the ear (2) via a first contact surface and wherein the at least one reference electrode (5) contacts the surface of the ear (2) via a second contact surface, wherein the second contact surface is at least 3 times as large as the first contact surface.

## Revendications

1. Dispositif (1) destiné à l'application d'un stimulus électrique transcutané sur la surface d'une portion de l'oreille humaine (2) combinée avec la diffusion d'un signal sonore dans le conduit auditif (G) de l'oreille (2), le dispositif (1) possédant au moins une tête à électrode (3) munie d'au moins une électrode (4, 5) servant à appliquer le stimulus électrique, le dispositif (1) possédant un haut-parleur (6) et un canal de diffusion (7) pour des signaux sonores dans le conduit auditif (G), le dispositif (1) comprenant en outre un appareil de commande (8) qui est configuré pour commander l'application du stimulus électrique et la diffusion de signaux sonores,
et l'au moins une électrode (4, 5) ou la tête à électrode (3) qui la supporte étant configurée pour pouvoir être disposée dans la cymba de la conque (Cy) de l'oreille (2),
**caractérisé en ce qu'**un étrier de maintien élastique (9) est disposé sur la tête à électrode (3), lequel est configuré pour envelopper l'oreille (2) et
**en ce que** le dispositif (1) possède en outre un générateur de vibrations (10) qui est configuré pour pouvoir appliquer des vibrations mécaniques sur une partie de crâne osseux, le générateur de vibrations (10) étant disposé sur l'étrier de maintien (9) ou intégré dans celui-ci.

2. Dispositif selon la revendication 1, **caractérisé en ce que** la tête à électrode (3) ainsi que l'au moins une électrode (4, 5) sont réalisées de manière réniforme et possède une surface d'appui principalement plane sur le côté qui fait face à l'oreille, la tête à électrode (3) venant en contact avec la surface de l'oreille (2) par le biais d'une surface de contact qui recouvre au moins 50 % de la surface de la cymba de la conque (Cy) de l'oreille (2).

3. Dispositif selon la revendication 1 ou 2, **caractérisé en ce que** le canal de diffusion (7) pour les signaux sonores est réalisé sous la forme d'un prolongement sur la tête à électrode (3).

4. Dispositif selon l'une des revendications 1 à 3, **caractérisé en ce que** des vibrations mécaniques peuvent être appliquées sur le rocher ou sur la mastoïde avec le générateur de vibrations (10).

5. Dispositif selon l'une des revendications 1 à 4, **caractérisé en ce que** l'appareil de commande (8) est configuré pour produire, de manière synchronisée, des stimulus électriques par le biais des électrodes (4, 5) et des signaux sonores par le biais du haut-parleur (6) et éventuellement des vibrations mécaniques par le biais du générateur de vibrations (10), la synchronisation comprenant des phases simultanées, des phases décalées dans le temps et des phases qui se chevauchent partiellement de stimulus, de signal et de vibrations.

6. Dispositif selon la revendication 5, **caractérisé en ce que** l'appareil de commande (8) possède un module électronique pour la production de stimulus électriques, de signaux sonores et de vibrations mécaniques.

7. Dispositif selon l'une des revendications 1 à 6, **caractérisé en ce que** l'au moins une électrode (4, 5) se compose au moins partiellement d'un matériau synthétique qui est muni de moyens servant à établir la conductivité électrique, les moyens servant à établir la conductivité électrique étant des particules électriquement conductrices qui sont enrobées dans le matériau synthétique ou les moyens servant à établir la conductivité électrique étant au moins une couche métallique électriquement conductrice appliquée galvaniquement sur un corps de base de l'électrode (4, 5).

8. Dispositif selon l'une des revendications 1 à 7, **caractérisé en ce qu'**au moins deux électrodes (4, 5) sont prévues, à savoir au moins une électrode de simulation (4) et une électrode de référence (5), l'au moins une électrode de simulation (4) venant en contact avec la surface de l'oreille (2) par le biais d'une première surface de contact et l'au moins une électrode de référence (5) venant en contact avec la surface de l'oreille (2) par le biais d'une deuxième surface de contact, la deuxième surface de contact étant au moins 3 fois plus grande que la première surface de contact.
